# EUROPÄISCHE PATENTANMELDUNG

(11) **EP 0 820 770 A2**
(43) Veröffentlichungstag der Anmeldung: **28.01.1998**
(21) Anmeldenummer: 97112167.8
(22) Anmeldetag: 16.07.1997
(51) Int. Cl.: A61K 31/675, A61K 31/385, A61K 31/375, A61K 31/355, A61K 31/07

(54) **Arzneimittel zur Behandlung von Neuropathien, das ein lipidlösliches Thiamin und ein Antioxidans enthält**

(30) Priorität: 24.07.1996 DE 19629803
(71) Anmelder: WÖRWAG PHARMA GmbH, 71034 Böblingen (DE)
(72) Erfinder: Wörwag, Fritz, Dr., 70192 Stuttgart (DE)
(74) Vertreter: Weller, Wolfgang, Dr.rer.nat.

(57) **Zusammenfassung**

Ein Arzneimittel zur Prophylaxe und Therapie von Neuropathien weist als Wirkstoffe zumindest ein lipidlösliches Thiamin und ein Antioxidans auf.

## Beschreibung

Die Erfindung betrifft Arzneimittel, die zumindest ein lipidlösliches Thiaminderivat enthalten, zur Behandlung von Neuropathien.

Ein derartiges Arzneimittel ist aus der DE 42 06 422 A1 bekannt.

Die lipidlöslichen Thiaminderivate werden zusammen mit den wasserlöslichen Thiaminen unter dem allgemeinen Begriff Vitamin B1 zusammengefaßt. Als Vitamin ist Thiamin ein lebensnotwendiger Wirkstoff, der vom Organismus nicht selbst synthetisiert werden kann und deshalb über den Nahrungsweg aufgenommen werden muß.

Die Thiamine sind vor allem für den Kohlenhydratstoffwechsel unentbehrlich. Die über die Nahrung aufgenommenen Thiamine werden im Körper in die biologisch wirksame Form, das Thiaminpyrophosphat (TPP), umgewandelt. Dabei ist das TPP ein essentieller Bestandteil von Enzymen, die am Abbau von Kohlenhydraten sowie an der Gewinnung von Energie und Stoffwechselzwischenprodukten (Intermediaten) aus Kohlenhydraten beteiligt sind. Weder die über die Nahrung aufgenommenen Thiamine noch die biologisch wirksame Form können vom Organismus gespeichert werden.

Aus der oben genannten DE 42 06 422 A1 geht hervor, daß Thiamine zur Behandlung von Neuropathien eingesetzt werden können. Neuropathien sind Nervenschädigungen, die in verschiedenen Formen vorkommen, bspw. als degenerative, toxische, metabolische oder ischämische Neuropathie, wobei die zuletzt genannte Form durch Durchblutungsstörungen zustande kommt. Unter dem Sammelbegriff Polyneuropathien werden nicht-entzündliche Erkrankungen mehrerer peripherer Nerven verstanden, deren Ursachen hauptsächlich chronischer Alkoholismus, Diabetes mellitus oder Schwermetallvergiftungen sind.

Bei der Therapie und Prophylaxe von Neuropathien wirken sich vor allem die lipidlöslichen (d.h. fettlöslichen) Thiamine, insbesondere die Allithiamine, günstig aus. Das chemische Grundgerüst der wasserlöslichen Thiamine weist einen positiv geladenen Thiazoliumring auf. Die lipidlöslichen Allithiamine unterscheiden sich strukturell grundsätzlich dadurch, daß sie in einer offenen Thiolform vorliegen. Dabei ist die Thiolgruppe mit einem apolaren Rest modifiziert, der entweder aliphatisch oder aromatisch sein kann.

In der DE 29 17 008 A1 ist ein Präparat beschrieben, in dem das lipidlösliche Thiamin Octotiamim zusammen mit Antioxidantien enthalten ist. Die Antioxidantien dienen ausschließlich der Stabilisierung des Octotiamins.

In dem Derwent-Abstract 93-365141/46 ist ein Vitaminpräparat beschrieben, das Succinsäureester oder ein Tokopherolsalz sowie Vitamin B1 und Ascorbinsäure enthält. Dieses Vitaminpräparat soll zur Behandlung von Menopausenbeschwerden und peripheren Durchblutungsstörungen eingesetzt werden.

Aus der EP 0 234 464 A1 ist eine Zusammensetzung zur Behandlung von "Katzenjammer", also zur Bekämpfung der Acetaldehyd-Toxizität, bekannt, die Ascorbinsäure, Cystein und Fursultiamin enthält. Als weitere lipidlösliche Thiamine, die der toxischen Wirkung des Acetaldehyds entgegenwirken, sind Prosultiamin und Octotiamin genannt.

Die besondere Bedeutung der Thiamine für den Stoffwechsel des Nervensystems liegt darin begründet, daß im Nervensystem ausschließlich Kohlenhydrate, nicht aber Fette oder Proteine als Energiequelle herangezogen werden können.

Daß eine Unterversorgung mit dem Vitamin Thiamin zu einer Erkrankung des Nervensystems führen kann, zeigt sich besonders drastisch bei der Thiaminmangelkrankheit Beriberi (Polyneuritis), bei der vor allem das periphere Nervensystem betroffen ist. Diese Krankheit äußert sich in Bewegungsstörungen bis hin zur Paralyse, die durch Nervenschädigungen vor allem in den Extremitäten zustande kommen.

Zur Therapie von Neuropathien können hohe Dosen an den gut resorbierbaren lipidlöslichen Thiaminen eingesetzt werden. Dennoch wurde festgestellt, daß manche Patienten, insbesondere solche mit diabetischer Polyneuropathie, therapieresistent sind, oder die Behandlung auch bei hoher Dosierung nicht zum gewünschten Ergebnis führt.

Es ist nun Aufgabe der vorliegenden Erfindung, ein Arzneimittel zu schaffen, das lipidlösliche Thiamine enthält, und mit dem Neuropathien effizienter therapiert werden können.

Erfindungsgemäß wird die Aufgabe dadurch gelöst, daß als Wirkstoff neben dem lipidlöslichen Thiamin Benfotiamin zumindest ein Antioxidans ("Radikalfänger") enthalten ist.

Im Organismus entstehen Radikale in der Atmungskette, bei der aus Intermediaten, die bei der Verstoffwechselung von Kohlenhydraten entstehen, Energie gewonnen wird. Dabei besteht die Atmungskette aus einer Reihe von Enzymkomplexen, die Metallionen enthalten. Diese sind in die verzweigten Membransysteme bestimmter intrazellulärer Organellen, den Mitochondrien, eingelagert.

In der Atmungskette werden einzelne Elektronen auf molekularen Sauerstoff übertragen, wobei teilweise reduzierte Zwischenprodukte entstehen, die als Radikale bezeichnet werden. Unter Radikalen versteht man somit Atome, Moleküle oder Ionen mit einem ungepaarten Elektron. Die bei der Atmungskette entstehenden Radikale sind vor allem das Peroxid-Anion und Alkyl-Radikale. Diese hochreaktiven, für den menschlichen Organismus sehr gefährlichen Radikale werden zwar normalerweise in den Enzymkomplexen der Atmungskette festgehalten, jedoch kommt es bei hohen Durchflußraten auch zu einer Freisetzung von Radikalen. Diese greifen essentielle Zellbestandteile wie die Erbsubstanz (DNA), Proteine, Steroide und vor allem Membranlipide an. Dadurch kann die Erbsubstanz verändert werden, Enzyme können inaktiviert, Hormone zerstört und die Struktur von Membranen verändert werden.

Zellen weisen eine Reihe von Entgiftungsmechanismen auf, mit Hilfe derer sie die zerstörerisch wirkenden Radikale inaktivieren können. Ein Beispiel dafür ist das Enzym Peroxid-Dismutase (SOD), das aus zwei Peroxid-Anionen Wasserstoffperoxid und Sauerstoff herstellen kann. Das ebenfalls giftige Wasserstoffperoxid kann durch das Enzym Katalase in Wasserstoff und Sauerstoff umgewandelt werden.

Ein weiterer Entgiftungsmechanismus der Zelle in das Enzym Peroxidase, bei dem ein radikalisches Alkyl-Peroxid zu Wasser und einem Alkohol reduziert wird. Diese Reaktion kann nur in Anwesenheit eines geeigneten Reduktionsmittels stattfinden.

An sich arbeiten die körpereigenen Entgiftungsmechanismen effizient. Es ist jedoch nicht sichergestellt, daß bei einem Mehranfall an Radikalen und Peroxiden die Entgiftungsreserve des Organismus ausreicht, insbesondere dann nicht, wenn diesem Zustand anhaltende pathologische Ursachen, wie bspw. Diabetes mellitus, zugrundeliegen. Der vermehrte oxidative Streß wird als eine der Ursachen der Degeneration von Nervenzellen angesehen. Diese führt zu Mißempfindungen und Schmerzen insbesondere an den Extremitäten, der sogenannten diabetischen Polyneuropathie.

Wie nun in einer weiter unten vorgestellten Studie belegt werden kann, wirkt sich eine Therapie von Neuropathien mit Benfotiamin und einem Antioxidans vorteilhafter aus als eine Therapie mit Benfotiamin alleine. Dabei verbesserten sich sowohl die objektiv meßbaren als auch die subjektiven Beschwerden der Patienten.

Ein Arzneimittel, das als Wirkstoffe Benfotiamin und zumindest ein Antioxidans enthält, hat den wesentlichen Vorteil, daß die erkrankten Nerven vor einer Schädigung durch Zellgifte wie Radikale und Peroxide geschützt werden können. Dabei ist ein entsprechend hoher Spiegel an Antioxidantien wichtig, um die anfallenden Radikale möglichst quantitativ unwirksam zu machen. Ein solch hoher Spiegel wird durch die Aufnahme von Antioxidantien über den Nahrungsweg alleine nicht erreicht, vor allem dann nicht, wenn die Resorption durch eine Stoffwechselerkrankung oder Alkoholmißbrauch ohnehin gestört sind. Daher ist es entsprechend der vorliegenden Erfindung angebracht, Antioxidantien zusätzlich zu den zur Therapie der Neuropathien eingesetzten lipidlöslichen Thiaminen zu verabreichen.

Somit wird die Aufgabe vollkommen gelöst.

Als Antioxidantien werden bevorzugt die α-Liponsäure, ein Tokopherol (Vitamin E-Gruppe), die Ascorbinsäure (Vitamin C), β-Carotin oder Selen bzw. eine Selenverbindung eingesetzt. Wie in der weiter unten ausgeführten Studie belegt wird, wirkt sich auch eine Kombination aus den Antioxidantien Vitamin C, Vitamin E, β-Carotin und Selen-Hefe besonders vorteilhaft aus.

Die α-Liponsäure hat den Vorteil, daß sie nicht nur eine Funktion als Radikalfänger ausübt, sondern zusammen mit ihrer reduzierten Form, der Dihydro-Liponsäure, ein ohnehin wichtiges Redoxpaar in der Zelle ist. Sie wird als Reaktionspartner bei Redoxreaktionen benötigt, die bei der Verstoffwechselung von Kohlenhydraten stattfinden.

In einer weiteren vorteilhaften Ausgestaltung der Erfindung wird ein Tokopherol (Vitamin E) als Antioxidans eingesetzt. Die Tokopherole mit ihrem wichtigsten Vertreter, dem α-Tokopherol, fangen vornehmlich Peroxide ab und schützen vor allem Lipidmembrane und die darin enthaltenen ungesättigten Fettsäuren. Darüber hinaus wirken die Tokopherole entzündungshemmend, indem sie die Synthese der bei der Entzündungsreaktion gebildeten Thromboxane und Prostacycline inhibieren. Diese entzündungshemmende Wirkung kann sich zusätzlich heilsam auf Neuropathien auswirken.

In einer weiteren vorteilhaften Ausgestaltung der Erfindung wird als Antioxidans die Ascorbinsäure (Vitamin C) eingesetzt.

Die Ascorbinsäure ist eines der wichtigsten Reduktionsmittel im menschlichen Körper und nimmt an der Entgiftung schädlicher Radikale vor allem durch ihre Beteiligung an der Peroxidase-Reaktion teil, durch die, wie oben schon erwähnt, Alkyl-Radikale entgiftet werden können. Dabei ist es wichtig, entsprechend hohe Dosen von Ascorbinsäure zu verabreichen, um auch in der Umgebung von Nervenzellen bzw. deren Membranen einen hohen Spiegel dieses wasserlöslichen Antioxidans zu erreichen.

Der Einsatz des Carotinoids β-Carotin als Antioxidans hat den Vorteil, daß es als Provitamin A eine für den menschlichen Organismus ohnehin essentielle Komponenten darstellt.

In einer bevorzugten Ausgestaltung der Erfindung wird ein lipidlösliches Thiamin zusammen mit einem Antioxidans und Selen (insbesondere als Selenhefe) oder einer Selenverbindung verabreicht. Selen ist als Spurenelement für den Menschen essentiell. Als Teil der sehr ungewöhnlichen Aminosäure Selenocystein ist das Selen im aktiven Zentrum des Enzyms Gluthation-Peroxidase enthalten. Die Gluthation-Peroxidase katalysiert eine essentielle Entgiftungsreaktion, indem sie mit Hilfe von Gluthation Wasserstoffperoxid und organische Peroxide reduziert. Darüber hinaus sollen Selenite die Wirkung von Tokopherolen steigern und an der Entgiftung von Schwermetallen beteiligt sein.

Wie in der weiter unten vorgestellten Studie festgestellt wurde, besteht eine besonders vorteilhafte Ausgestaltung der Erfindung in einem Arzneimittel, das als Wirkstoff Benfotiamin und α-Liponsäure enthält. Hier liegt als erster Wirkstoff das günstig resorbierbare, lange im Körper verbleibende Benfotiamin vor und als zweiter Wirkstoff das Antioxidans α-Liponsäure, das sehr effizient bei der Entgiftung von Radikalen wirksam ist.

Wie in der Studie außerdem belegt werden konnte, ist es weiter vorteilhaft, als Wirkstoff Benfotiamin in Verbindung mit Vitamin C, Vitamin E, β-Carotin und Selenhefe einzusetzen.

Das molare Verhältnis des lipidlöslichen Thiamins zum Antioxidans kann von 100:1 bis 1:100, vorzugsweise im Bereich von 50:1 und 1:50, oder höchst vorzugsweise im Bereich von 1:1 bis 1:10 liegen.

Die Erfindung betrifft ferner die Verwendung eines Arzneimittels, das als Wirkstoff ein lipidlösliches Thiamin und zumindest ein Antioxidans enthält, zur Behandlung von Neuropathien, insbesondere von Polyneuropathien oder Neuropathien, die in Verbindung mit Stoffwechselerkrankungen wie der Diabetes mellitus, Mangelernährung oder Alkoholmißbrauch sowie exogenen toxischen Stoffen stehen.

In einer bevorzugten Ausgestaltung werden unter den lipidlöslichen Thiaminderivaten die Allithiamine ausgewählt, die nach oraler Einnahme erheblich besser intestinal resorbiert werden als die wasserlöslichen Thiamine (R. Bitsch und I. Bitsch, "Lipidlösliche Thiaminderivate", Deutsche Apothekerzeitung, Nr. 2, 1989, Seiten 65-68). Daher werden bei oraler Verabreichung von Allithiaminen wesentlich höhere Thiaminspiegel in Blut und Gewebe erreicht als bei der Gabe gleicher Mengen wasserlöslicher Thiamine. Außerdem werden Allithiamine wesentlich besser im Körper zurückgehalten, d.h. in weniger großen Mengen ausgeschieden als andere Thiaminderivate.

Unter den Allithiaminen sind die aromatischen Derivate wie das Benfotiamin, Bentiamin und Octotiamin sowie das nicht aromatische Fursultiamin zu bevorzugen, da sie im Gegensatz zu den aliphatischen Vertretern nicht geruchsintensiv sind und zudem eine höhere Hitzestabilität und eine vermehrte Resistenz gegenüber Thiaminasen aufweisen. Außerdem weisen die Allithiamine gegenüber dem Thiaminchloridhydrochlorid , einem wasserlöslichen Thiamin, eine vergleichsweise geringere Toxizität auf und sind so therapeutisch besser einsetzbar.

In einer besonders vorteilhaften Ausgestaltung der Erfindung wird das Benfotiamin eingesetzt, das eine geringe Toxizität aufweist und zudem die Bluthirnschranke passieren kann.

Es versteht sich, daß die vorstehend genannten und die nachstehend noch zu erläuternden Merkmale nicht nur in den jeweils angegebenen Kombinationen, sondern auch in anderen Kombinationen oder in Alleinstellung verwendbar sind, ohne den Rahmen der vorliegenden Erfindung zu verlassen.

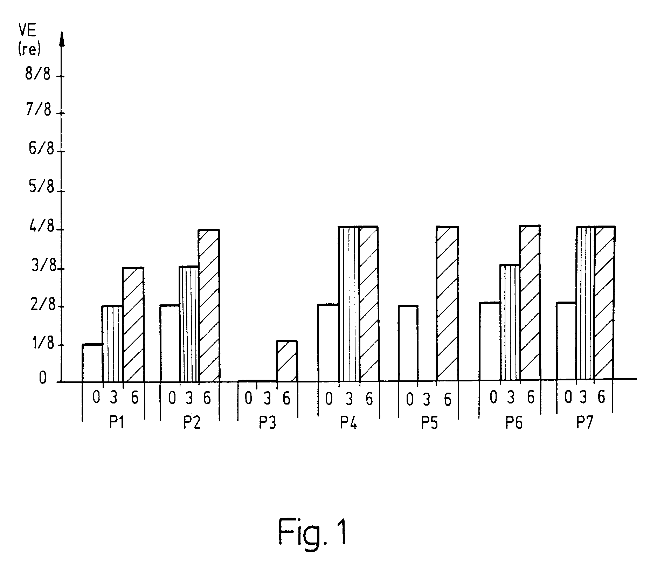

Die Erfindung wird nachfolgend in Verbindung mit der beiliegenden Zeichnung anhand einer Studie mit insgesamt sieben Neuropathie-Patienten näher erläutert.

Alle Patienten wurden zunächst über längere Zeiträume hinweg (ca. zwei Monate) nur mit Benfotiamin behandelt, wobei keine erhebliche Besserung des Krankheitsbildes beobachtet werden konnte. Diese Patienten wurden anschließend jeweils mit Benfotiamin und zusätzlich mit einem oder mehreren Antioxidantien behandelt.

Unter anderem wurde das Vibrationsempfinden im rechten großen Zeh im sogenannten Riedel-Seyfert-Test gemessen.

Mit diesem Test kann das Ausmaß der durch Neuropathien hervorgerufenen Empfindungsstörungen gemessen werden. Der Test besteht darin, daß das Vibrationsempfinden (VE) an oberflächlich liegenden Körperteilen mit Hilfe einer skalierten Stimmgabel auf einer Skala von 0 bis 8/8 gemessen wird. So erhält man einen objektivierbaren neurologischen Parameter, mit Hilfe dessen der Therapieerfolg des eingesetzten Arzneimittels bestimmt werden kann.

In der einzigen beiliegenden Zeichnung sind die Ergebnisse des Riedel-Seyfert-Tests dargestellt.

Die Ergebnisse nach der Behandlung mit Benfotiamin alleine sind als weißer Balken dargestellt. Die Ergebnisse einer Behandlung mit Benfotiamin und mit einem Antioxidans nach drei Wochen sind als längsschraffierter Balken und die Ergebnisse nach einer sechswöchigen Behandlung sind als querschraffierter Balken dargestellt.

### Patient 1: Therapie mit Benfotiamin und α-Liponsäure

Eine 1940 geborene Patientin (P1) litt seit 20 Jahren an Typ I-Diabetes mellitus. Bei ihr hatte sich seit ca. 16 Monaten eine diabetische Polyneuropathie manifestiert. Die Hauptbeschwerden dieser Patientin waren neuropathische Schmerzen sowie Gefühlsstörungen in beiden Füßen.

Sie wurde nach einer anfänglichen Monotherapie mit Benfotiamin mit 150 mg Benfotiamin und 2 x 600 mg α-Liponsäure täglich über einen Zeitraum von sechs Wochen behandelt.

Bei dieser Patientin konnten die Gefühlsstörungen in den Füßen wesentlich gebessert werden und ihr Allgemeinzustand verbesserte sich deutlich. Darüber hinaus waren keine Nebenwirkungen des erfindungsgemäßen Arzneimittels zu beobachten.

Das Vibrationsempfinden (VE) im rechten großen Zeh steigerte sich von 1/8 nach der Monotherapie mit Benfotiamin auf 2/8 nach drei Wochen Behandlung mit Benfotiamin und α-Liponsäure. Nach sechs Wochen dieser Therapie war das Vibrationsempfinden auf 3/8 angestiegen.

Insgesamt zeigte die Behandlung der schmerzhaften diabetischen Polyneuropathie dieser Patientin einen sehr guten Therapieerfolg.

### Patient 2: Therapie mit Benfotiamin und α-Liponsäure

Eine 1945 geborene Patientin (P2) litt seit zwölf Jahren an Typ I-Diabetes mellitus und klagte über eine seit sechs Monaten bestehende diabetische Polyneuropathie. Diese äußerte sich in Schmerzen in beiden Füßen und Unterschenkeln.

Nach einer anfänglichen Therapie mit Benfotiamin alleine wurde die Patientin mit 150 mg Benfotiamin und mit 2 x 600 mg α-Liponsäure täglich über einen Zeitraum von sechs Wochen therapiert.

Über den Beobachtungszeitraum hinweg kam es zu einer deutlichen subjektiven Besserung der neuropathischen Beschwerden, was sich in einem Nachlassen der Schmerzen und in einem verbesserten Allgemeinzustand ausdrückte.

Das Vibrationsempfinden dieser Patientin steigerte sich von 2/8 nach der Monotherapie mit Benfotiamin auf 3/8 nach drei Wochen Therapie mit dem erfindungsgemäßen Arzneimittel und auf 4/8 nach sechs Wochen dieser Therapie.

Auch bei dieser Patientin war also durch eine kombinierte Gabe von Benfotiamin und einer antioxidativ wirkenden Verbindung eine erfolgreiche Behandlung der Polyneuropathie möglich.

### Patient 3: Therapie mit Benfotiamin und α-Liponsäure

Bei einem 1931 geborenen Patienten (P3), der seit sechs Monaten an Typ II-Diabetes mellitus litt, wurde eine diabetische Polyneuropathie diagnostiziert. Der Patient hatte starke Schmerzen in beiden Füßen, eine Gangunsicherheit und ein Taubheitsgefühl in beiden Füßen.

Auch dieser Patient wurde nach der anfänglichen Monotherapie mit Benfotiamin über insgesamt sechs Wochen hinweg mit 150 mg Benfotiamin und 2 x 600 mg α-Liponsäure täglich behandelt.

Nach der Therapie gingen die Schmerzen des Patienten nach seiner subjektiven Bewertung von stark auf mittel zurück und die Gefühlstörungen verbesserten sich.

Bei diesem Patienten lag das Vibrationsempfinden nach der Monotherapie mit Benfotiamin bei 0, auch nach drei Wochen Therapie mit Benfotiamin und α-Liponsäure war keine Änderung festzustellen. Nach einer sechswöchigen Therapie mit Benfotiamin und α-Liponsäure war dagegen ein Anstieg des Vibrationsempfindens auf 1/8 zu verzeichnen.

Insgesamt ließ sich auch bei diesem Patienten eine Verbesserung der neuropathischen Beschwerden durch das erfindungsgemäße Arzneimittel erzielen.

### Patient 4: Therapie mit Benfotiamin und Ascorbinsäure (Vitamin C)

Ein 1940 geborener Patient (P4) litt seit neun Jahren an Typ I-Diabetes mellitus und seit sechs Monaten an einer diabetischen Polyneuropathie. Dieser Patient klagte vor allen über Schmerzen in beiden Vorfüßen.

Nach Therapie mit Benfotiamin alleine wurde er mit 150 mg Benfotiamin und 2 x 500 mg Ascorbinsäure (Vitamin C) täglich therapiert.

Innerhalb des Therapiezeitraums von sechs Wochen kam es zu einer deutlichen Besserung der subjektiv-neuropathischen Beschwerden.

Das Vibrationsempfinden am rechten großen Zeh dieses Patienten verbesserte sich von 2/8 nach der Monotherapie auf 4/8 nach bereits drei Wochen Behandlung mit Benfotiamin und Ascorbinsäure. Auch nach sechs Wochen wurde ein Wert von 4/8 für das Vibrationsempfinden gemessen. Auch hier waren keine Nebenwirkungen des Arzneimittels zu verzeichnen.

Insgesamt konnte bei diesem Patienten die Gabe von Benfotiamin in Kombination mit Ascorbinsäure (Vitamin C) erfolgreich zur Therapie einer diabetischen Polyneuropathie eingesetzt werden.

### Patient 5: Therapie mit Benfotiamin und α-Tokopherol

Ein 1936 geborener, seit 14 Jahren an Typ I-Diabetes mellitus leidender Patient (P5) zeigte seit 14 Monaten Zeichen einer diabetischen Polyneuropathie. Diese äußerte sich in einem Taubheitsgefühl in den Zehen sowie in Schmerzen in beiden Füßen.

Nach anfänglicher Therapie mit Benfotiamin alleine wurde er über sechs Wochen hinweg mit 150 mg Benfotiamin und 2 x 600 mg α-Tokopherol-Acetat behandelt.

Nach sechs Wochen Therapie war eine Besserung der neuropathischen Schmerzen sowie eine Steigerung des Vibrationsempfindens am rechten großen Zeh von 2/8 auf 4/8 zu verzeichnen, wobei hier für den Zeitpunkt von drei Wochen kein Wert vorliegt.

Insgesamt zeigte bei diesem Patienten die Therapie von Benfotiamin in Verbindung mit einem Tokopherol eine Besserung der neuropathischen Beschwerden.

### Patient 6: Therapie mit Benfotiamin, Ascorbinsäure (Vitamin C), α-Tokopherol, β-Carotin und Selenhefe

Ein 1940 geborener Patient (P6) litt seit 23 Jahren an Typ I-Diabetes mellitus und seit neun Monaten an einer diabetischen Polyneuropathie.

Nach anfänglicher Monotherapie mit Benfotiamin wurde dieser Patient mit täglich 150 mg Benfotiamin sowie jeweils 3 x täglich 225 mg Ascorbinsäure Ascorbinsäure, 36 mg α-Tokopherol, 18 mg β-Carotin sowie 30 mg Selenhefe behandelt.

Nach einem Behandlungszeitraum von sechs Wochen wurden die neuropathischen Schmerzen dieses Patienten deutlich besser.

Das Vibrationsempfinden, das nach Monotherapie einen Wert von 2/8 aufwies, stieg bereits nach drei Wochen auf einen Wert von 4/8 an. Der verbesserte Wert blieb auch nach sechs Wochen Therapie mit dem erfindungsgemäßen Arzneimittel bestehen.

Bei diesem Patienten zeigte sich ein therapeutischer Erfolg unter der kombinierten Gabe von Benfotiamin, Ascorbinsäure, α-Tokopherol, β-Carotin und Selenhefe.

### Patient 7: Therapie mit Benfotiamin, Ascorbinsäure, α-Tokopherol, β-Carotin und Selenhefe

Eine 1949 geborene, seit 13 Jahren an Typ I-Diabetes mellitus leidende Patientin (P7) wies seit acht Monaten Zeichen einer diabetischen Polyneuropathie auf. Sie litt an mittleren neuropathischen Schmerzen sowie Sensibilitätsstörungen in beiden Füßen, die subjektiv als sehr unangenehm empfunden wurden.

Diese Patientin wurde nach anfänglicher Monotherapie mit Benfotiamin für insgesamt sechs Wochen mit einer Kombination aus Benfotiamin (150 mg täglich) sowie jeweils 3 x täglich 225 mg Ascorbinsäure, 36 mg α-Tokopherol, 18 mg β-Carotin sowie 30 mg Selenhefe behandelt.

Im Laufe der Therapie besserten sich die subjektiv-neuropathischen Beschwerden dieser Patientin erheblich.

Bereits nach drei Wochen Therapie mit dem erfindungsgemäßen Arzneimittel war das Vibrationsempfinden am rechten großen Zeh von 2/8 auf 4/8 angestiegen. Dieser Wert blieb auch nach sechs Wochen Therapie erhalten.

Ein sehr positiver Effekt der Therapie bei dieser Patientin war außerdem, daß sie weniger Schmerzmittel einnehmen mußte.

Insgesamt zeigte hier die Behandlung mit Benfotiamin in Verbindung mit Antioxidantien eine gute therapeutische Beeinflußbarkeit, die noch dadurch unterstrichen wird, daß die Schmerzmedikation deutlich vermindert werden konnte.

### Zuammenfassende Betrachtung

Zusammenfassend ergibt sich aus der hier vorgestellten Studie mit sieben an Polyneuropathien leidenden Patienten, daß bei allen Patienten, bei denen zunächst eine wesentliche Besserung durch die Monobehandlung mit Benfotiamin ausgeblieben war, durch die Gabe von Benfotiamin in Verbindung mit einem Antioxidans ein guter therapeutischer Erfolg erzielt werden konnte. Das Vibrationsempfinden in den Zehen der Patienten steigerte sich unter die Therapie mit Benfotiamin und einem Antioxidans um durchschnittlich 50 %, was für eine erhebliche Besserung der Sensibilitätsstörungen spricht. Außerdem war bei allen Patienten eine Verminderung der Schmerzen und eine Verbesserung des Gesamtzustandes zu verzeichnen. Bei einigen Patienten konnten zudem Taubheitsgefühle in den Extremitäten erfolgreich bekämpft werden. In einem Fall konnte sogar die Schmerzmedikation vermindert werden. Nebenwirkungen der Therapie waren in keinem Fall zu verzeichnen.

## Patentansprüche

1. Arzneimittel, enthaltend Benfotiamin, gekennzeichnet durch einen zusätzlichen Gehalt an zumindest einem Antioxidans als Wirkstoff.

2. Arzneimittel nach Anspruch 1, dadurch gekennzeichnet, daß das Antioxidans ausgewählt ist aus der Gruppe bestehend aus α-Liponsäure, den Tokopherolen (Vitamin E-Gruppe), der Ascorbinsäure (Vitamin C) und dem β-Carotin.

3. Arzneimittel nach Anspruch 1 oder 2, gekennzeichnet durch einen zusätzlichen Gehalt an Selen oder zumindest einer Selenverbindung.

4. Arzneimittel nach einem der Ansprüche 1 bis 3, dadurch gekennzeichnet, daß als Antioxidans α-Liponsäure enthalten ist.

5. Arzneimittel nach einem der Ansprüche 1 bis 4, dadurch gekennzeichnet, daß als Antioxidans α-Tokopherol, Ascorbinsäure, β-Carotin und Selen oder eine Selenverbindung enthalten sind.

6. Arzneimittel nach einem der Ansprüche 1 bis 5, dadurch gekennzeichnet, daß Benfotiamin und das Antioxidans in molaren Verhältnissen im Bereich von
100:1 bis 1:100, vorzugsweise im Bereich von 50:1 bis 1:50, höchst vorzugsweise im Bereich von 1:1 bis 1:10 vorliegen.

7. Verwendung eines Stoffgemischs, das ein lipidlösliches Thiamin und einen zusätzlichen Gehalt an zumindest einem Antioxidans aufweist, zur Herstellung eines Arzneimittels zur Behandlung von Neuropathien.

8. Verwendung eines Stoffgemischs, das ein lipidlösliches Thiamin und einen zusätzlichen Gehalt an zumindest einem Antioxidans aufweist, zur Herstellung eines Arzneimittels zur Behandlung von Polyneuropathien und Neuropathien, die in Verbindung mit Stoffwechselerkrankungen, Mangelernährung, exogenen toxischen Stoffen und Alkoholmißbrauch stehen.

9. Verwendung eines Stoffgemischs nach Anspruch 7 oder 8, bei dem als lipilösliches Thiamin ein Allithiamin verwendet wird.

10. Verwendung eines Stoffgemischs nach Anspruch 9, bei dem das Allithiamin ausgewählt ist aus der Gruppe bestehend aus Benfotiamin, Bentiamin, Fursultiamin oder Octotiamin.

11. Verwendung eines Stoffgemischs nach einem der Ansprüche 7 bis 10, bei dem das Antioxidans ausgewählt ist aus der Gruppe bestehend aus α-Liponsäure, den Tokopherolen (Vitamin E-Gruppe), der Ascorbinsäure (Vitamin C) und dem β-Carotin.

12. Verwendung eines Stoffgemischs nach einem der Ansprüche 7 bis 11, bei dem ein zusätzlicher Gehalt an Selen oder zumindest einer Selenverbindung verwendet wird.

13. Verwendung eines Stoffgemischs nach einem der Ansprüche 7 bis 12, bei dem als Allithiamin Benfotiamin und als Antioxidans α-Liponsäure verwendet wird.

14. Verwendung eines Stoffgemischs nach einem der Ansprüche 7 bis 13, bei dem als Allithiamin Benfotiamin und als Antioxidantien α-Tokopherol, Ascorbinsäure, β-Carotin und Selen oder eine Selenverbindung verwendet werden.
